# EUROPEAN PATENT APPLICATION

(11) **EP 0 532 100 A1**
(43) Date of publication of application: **17.03.1993**
(21) Application number: 92202703.2
(22) Date of filing: 07.09.1992
(51) Int. Cl.: C07D 491/056, A61K 31/40

(54) **Benzodioxinopyrrole derivative as alpha 2-adrenoreceptor antagonist**

(30) Priority: 12.09.1991 GB 9119466
(71) Applicant: GLAXO GROUP LIMITED, Greenford, Middlesex UB6 0NN (GB)
(72) Inventor: Cherry, Peter Clive, Glaxo Group Res. Ltd., Greenford, Middlesex UB6 0HE (GB); Kitchin, John, Glaxo Group Res. Ltd., Greenford, Middlesex UB6 0HE (GB); Pipe, Adrian John, Glaxo Group Res. Ltd., Greenford, Middlesex UB6 0HE (GB)
(74) Representative: James, Stephen Richard, Dr.

(57) **Abstract**

The present invention provides the optical isomers of the compound I, namely the compounds
(+)-3aR-trans-5-fluoro-2,3,3a,9a-tetrahydro-1H-[1,4]-benzodioxino[2,3-c]pyrrole
or
(-)-3aS-trans-5-fluoro-2,3,3a,9a-tetrahydro-1H-[1,4]-benzodioxino[2,3-c]pyrrol
or the physiologically acceptable salts or hydrates thereof. The claimed compounds are for use in medicine, especially for the treatment of conditions that are subject to amelioration by compounds having selective α₂-adrenoreceptor antagonistic activity.

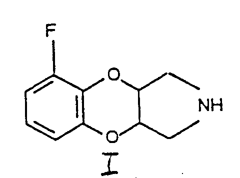

## Description

The present invention relates to optical isomers of a known 1,4-benzodioxino[2,3-c]pyrrole derivative, to a process for their preparation, to pharmaceutical compositions containing them and to their use in medicine.

GB Patent Publication No. 2157691A describes the preparation of the racemic form of the compound of formula I. 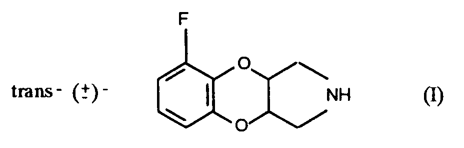
This compound being (±)- trans-5-fluoro-2,3,3a,9a-tetrahydro-1H-[1,4]-benzodioxino[2,3-c]pyrrole (hereinafter referred to as "Compound A"). A preferred form of the compound is the hydrochloride, particularly in hydrated form, for example the hemihydrate.

We now provide both optical isomers of Compound A.

According to one aspect of the present invention there is provided an optically active compound selected from (+)- 3aR-trans-5-fluoro-2,3,3a,9a-tetrahydro-1H-[1,4]-benzodioxino[2,3-c]-pyrrole and (-)- 3aS-trans-5-fluoro-2,3,3a,9a-tetrahydro-1H-[1,4]-benzodioxino[2,3-c]pyrrole, and the physiologically acceptable salts and hydrates thereof.

The isomers are referred to hereinafter as the "(R)-isomer of Compound A" and the "(S)-isomer of Compound A".

The compounds of the present invention are selective α₂- adrenoreceptor antagonists of interest in the treatment or prevention of, for example, migraine, thrombosis, diabetes, obesity, hypertension, constipation, paralytic ileus, senile dementia and, in particular, for the treatment of depression or sexual dysfunction.

In the present specification, the sexual dysfunction may be of the male or female variety. In the former case, male sexual dysfunction incorporates male erectile disorder (erectile inadequacy) and inhibited male orgasm. In the case of female sexual dysfunction, this includes sexual arousal disorder and inhibited female orgasm.

The test for determining the α₂-adrenoreceptor antagonist action is based on the ability to prevent the action of a selective α₂-adrenoreceptor agonist such as clonidine or 5-bromo-N-(4,5-dihydro-1H-imidazol-2-yl)-6- quinoxalinamine, [R-(R*R*)]-2,3-dihydroxybutanedioate (UK 14304-18) on the rat field stimulated vas deferens preparation.

Clonidine and UK 14304-18 inhibit the twitch response of the rat isolated vas deferens to low frequency motor nerve stimulation. This inhibition is a consequence of activation of presynaptic adrenoreceptors of the α₂-type. Antagonism of the effect of clonidine or UK 14304-18 is quantified by measuring the parallel shift to the right of the inhibitory α₂-adrenoreceptor agonist log₁₀(concentration)/response curve in the presence of increasing concentrations of the antagonist. Potency and competitiveness of antagonism are determined by the method of Arunlakshana & Schild(Br.J.Pharmac. 1959, 14, 48-58).

The α-adrenoreceptor-type selectivity of the presently claimed compounds is similarly assessed by measuring the ability to produce a parallel shift to the right of the log₁₀ (concentration)/response curve for the α₁-adrenoreceptor agonist phenylephrine. The α₁-adrenoreceptor-mediated responses of phenylephrine measured were contractions of the rat isolated anococcygeus muscle (Leighton, Butz & Parameter, Eur.J.Pharmac., 1979 58 27-38).

In a further or alternative aspect of the present invention, therefore, we provide the (R)-isomer of Compound A or the (S)- isomer of Compound A or a physiologically acceptable salt or hydrate thereof for use in medicine, more particularly for use in the treatment of conditions which may be ameliorated by administration of a selective α₂-adrenoreceptor antagonist.

In another aspect, the invention further provides a method for the treatment of a condition subject to amelioration in a mammal including a human by administration of a selective α₂- adrenoreceptor antagonist, comprising administration of an effective amount of the (R)-isomer of Compound A, or the (S)-isomer of Compound A, or a physiologically acceptable salt or hydrate thereof.

There is also provided in a further or alternative aspect, use of the (R)-isomer of Compound A or the (S)-isomer of Compound A, or a physiologically acceptable salt or hydrate thereof, for the manufacture of a medicament for the treatment of a condition which may be ameliorated by administration of a selective α₂-adrenoreceptor antagonist.

It is to be understood that references herein to treatment may extend to prophylaxis as well as the treatment of established conditions.

Suitable physiologically acceptable salts disclosed are the acid addition salts formed with inorganic acids for example hydrochlorides, hydrobromides, phosphates and sulphates, and with organic acids, for example, citrates, tartrates, acetates, maleates, tosylates, embonates and succinates.

In a particularly preferred embodiment of the present invention, the compound used is the hydrochloride salt, particularly in hydrated form, for example as the hemihydrate.

It is possible that a compound of the invention may be administered to a patient as the raw chemical, but it is preferable to present the active ingredient as a pharmaceutical formulation, especially in unit dosage form.

The invention accordingly provides a pharmaceutical formulation comprising the (R)-isomer of Compound A or the (S)-isomer of Compound A or a physiologically acceptable salt or hydrate thereof together with one or more physiologically acceptable carriers, and optionally, other therapeutic and/or prophylactic ingredients. The carrier(s) must be "acceptable" in the sense of being compatible with the other ingredients of the formulation and not deleterious to the recipient thereof.

A compound according to the invention may be formulated in a conventional manner using one or more pharmaceutically acceptable carriers or excipients for administration by any convenient route, for example for oral, rectal or parenteral administration. A compound according to the invention may be conveniently formulated for parenteral or preferably oral administration.

For oral administration the pharmaceutical composition may take the form of, for example, tablets or capsules prepared by conventional means with pharmaceutically acceptable excipients such as binding agents (for example pregelatinised maize starch, polyvinylpyrrolidone or hydroxypropyl methylcellulose); fillers (for example lactose, microcrystalline cellulose or calcium phosphate); lubricants (for example magnesium stearate, talc or silica); disintegrants (for example potato starch or sodium starch glycollate); or wetting agents (for example sodium lauryl sulphate.) The tablets may be coated by methods well known in the art. Liquid preparations for oral administration may take the form of, for example, solutions, syrups or suspensions, or they may be presented as a dry product for constitution with water or other suitable vehicles before use. Such liquid preparations may be prepared by conventional means with pharmaceutically acceptable additives such as suspending agents (for example sorbitol syrup, methyl cellulose or hydrogenated edible fats); emulsifying agents (for example lecithin or acacia); non-aqueous vehicles (for example methyl or propyl-p-hydroxybenzoates or sorbic acid).

A compound according to the invention may be formulated for parenteral administration by injection, conveniently intravenous or subcutaneous injection, for example by bolus injection or continuous intravenous infusion. Formulation for injection may be presented in unit dosage form, for example, in ampoules or in multi-dose containers with added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, for example sterile pyrogen-free water, before use.

Compositions for rectal administration may be in the form of suppositories using a conventional suppository excipient.

It will be appreciated that the precise dose administered will depend on the age and condition of the patient, the particular form of the compound used, and the frequency and route of administration. compound according to the invention may be administered in single or divided doses and may be administered one or more times, for example 1 to 4 times, per day.

A proposed daily dose of a compound according to the invention or administration to man is 0.01 to 10mg/kg, for example 0.05 to mg/kg. The daily dose may conveniently be administered in unit dosage form, each containing for example 0.01 to 3mg/kg of active ingredient.

The compounds of the invention may be prepared according to the general methods described in GB Patent Publication No. 2201414A from starting materials having the desired stereochemical configuration. According to a first example (A), a compound according to the invention may be prepared by amination of an optically active trans-benzodioxin derivative of general formula (II). 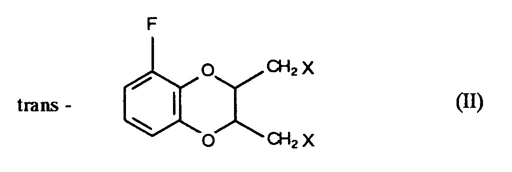
where X is a leaving group such as a halogen atom, (e.g. chlorine. bromine or iodine), or a hydrocarbylsulphonyloxy group (e.g. methylsulphonyloxy)] with ammonia, aqueous ammonia or an amine of formula RNH₂ where R is a nitrogen protecting group.

The amination reaction is conveniently effected at an elevated temperature e.g. under reflux or in a sealed tube at e.g 110⁰C preferably in the presence of a suitable base e.g sodium hydride or an alkali metal hydroxide such as sodium hydroxide, or in the presence of an excess of the amine RNH₂ optionally in the presence of solvent such as a chlorinated hydrocarbon e.g. chloroform or another e.g. dioxan or an alcohol e.g. ethanol. Optional removal of an arylmethyl protecting group may be carried out, for example, by hydrogenolysis or, where appropriate, under acidic conditions, as described below.

According to another example (B) , a compound according to the invention may be prepared by deprotection of a corresponding optically active compound where the nitrogen atom of the pyrrole ring is protected by a nitrogen protecting group. Suitable protecting groups include, for example, arylmethyl and acyl groups. Conventional deprotection procedures may be used. For example, where appropriate an arylmethyl group (e.g. benzyl) may be removed by hydrogenolysis using, for example, hydrogen in the presence of a catalyst, such as platinum or palladium on a support (e.g. charcoal) in a solvent such as an alcohol e.g. methanol. Alternatively, where appropriate, an arylmethyl group (e.g. trityl or bis(4-methoxyphenyl)methyl) may be removed under acidic conditions using for example an acid such as trifluoroacetic acid, formic acid, hydrochloric acid or hydrobromic acid. Acyl groups may be removed by hydrolysis using an acid such as a mineral acid or a base such as an alkali metal hydroxide as appropriate. The protected starting materials for this process may be prepared using standard methods for the protection of amines, for example as described by Theodora Green in "Protective Groups in Organic Synthesis" , John Wiley, 1981.
The intermediate isomers of general formula (II) may be prepared by reaction of a corresponding optically active diol of formula (III) 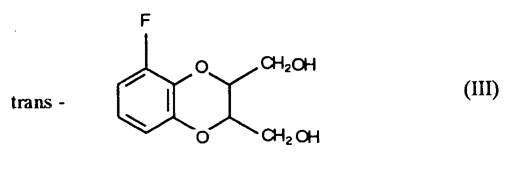
with a halide of formula X₁A (where X₁ is a hydrocarbylsulphonyl group e.g. methylsulphonyl and A is a halogen atom e.g. a chlorine atom) in the presence of a base e.g. triethylamine in a solvent such as dichloromethane, or with a halogenating agent such as thionyl chloride, phosphorus tribromide or hydrogen iodide.

A diol (III) may be prepared by reduction of a corresponding optically active dibenzyl ether of formula (IV) 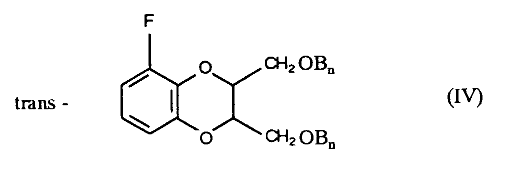
(where Bn represents benzyl) using hydrogen and palladium on charcoal with a solvent e.g. ethanol.

Alternatively, an ether of formula (IV) may be treated with a Lewis acid, such as aluminium chloride, in a solvent such as toluene and in the presence of a suitable carbonium ion scavenger e.g. anisole to yield a diol of formula (III)
An optically active compound of formula (IV) may be prepared by heating an optically active bis-tosylate (V) or (VI) 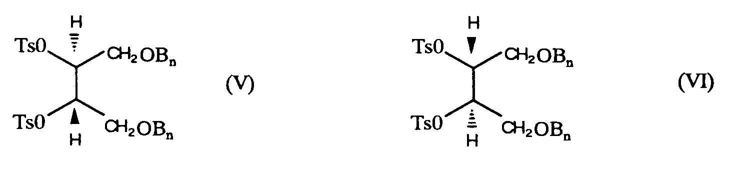
(where Ts represents 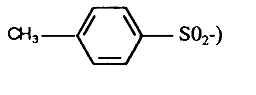
with a catechol of formula (VII) 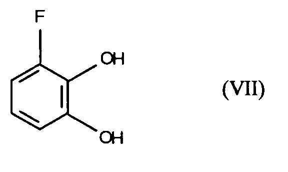
in acetonitrile or dimethylformamide containing caesium fluoride or cesium carbonate.

The intermediates of formula (VII) are known compounds and may be prepared by standard methods of benzene ring substitution (cf. for example Barton, Linnell & Senior, Quat.J.Pharmacy & Pharmac., 1945, 18, 41-47 and Ladd & Weinstock, J.Org.Chem. 1981, 46, 203-206).

The bis-tosylates of formulae (V) and (VI) may be prepared by reaction of the known dibenzyl threitols of formulae (VIII) and (IX) 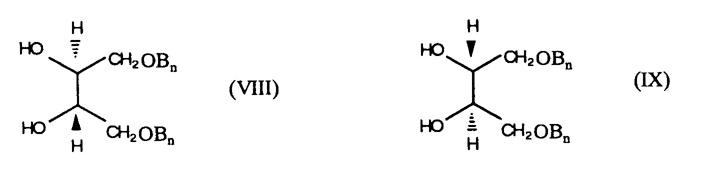
with a 4-toluenesulphonyl chloride in pyridine.

The present optical isomers may also be prepared by resolution of a mixture of the optical isomers by conventional methods, e.g. by salt formation with an optically active acid followed by separation of the resulting diastereoisomeric salts, e.g. by fractional crystallisation. Alternatively, resolution may be effected at any suitable intermediate stage.

The following examples illustrate the invention. All temperatures are in ⁰C. "Petroleum ether" refers to the fraction boiling at 60-80⁰ unless otherwise stated. "Dried" refers to drying of the solvent over magnesium sulphate unless otherwise stated. "IMS" and "IPE" represent industrial methylated spirits and di-isopropyl ether respectively. In the following "EtOH", "CHCl₃ and "DMSO" represent ethanol, chloroform and dimethyl sulphoxide respectively.

### Intermediate 1

### 1,4-Di-O-benzyl-L-threitol

This compound may be prepared by the method described by N. Ando et al, Synthesis, 1978, 688.

### Intermediate 2

### [2S-(R*,R*)]-(-)-1,4-Bis(phenylmethoxy)-2,3-butanediol, bis(4-methylbenzenesulphonate)

To a solution of 1,4-di-0-benzyl-L-threitol (4g) in pyridine (50ml), cooled in ice, was added toluene-4-sulphonyl chloride (6g) and the mixture was stirred at 20⁰ for 4 days. The mixture was poured into ethyl acetate (300 ml) and the solution was washed successively with 2M hydrochloric acid (3 x 160 ml), saturated sodium bicarbonate solution (3 x 120 ml), and water (3 x 120 ml). The solution was dried and evaporated giving a solid which, after trituration with ether (50ml), was collected, washed with ether and dried to give the title compound (5.64g),m.p. 125-127⁰.
[α]_{D}-19.6(c 0.89, CHCl₃).

### Intermediate 3

### (+)-trans-5-Fluoro-2,3-dihydro-2,3-bis[(phenylmethoxy)methyl]-1,4-benzodioxin

3-Fluorobenzene-1,2-diol (4.0g) and [2S-(R*,R*)]-(-)-1,4- bis(phenylmethoxy)-2,3-butanediol, bis(4-methylbenzenesulphonate) (22.88g) were suspended in dimethylformamide (100ml) and the mixture was stirred under nitrogen for 0.75h. Anhydrous caesium carbonate (11.19g) was then added and the mixture was refluxed under nitrogen for 24h. The mixture was allowed to stand at ambient temperature for 2 days, diluted with water (250ml) and the black suspension was extracted with IPE (1 x 300ml and 2 x 250ml). The combined extracts were washed with M hydrochloric acid (300ml), 30% brine solution (100ml) and dried. Evaporation in vacuo gave a dark brown oil (11.63g) which was chromatographed on Sorbsil (400g), eluting with petroleum ether(40-60)-ethyl acetate (9:1). Fractions containing Intermediate 3 were combined and evaporated in vacuo to give an oil (7.296g).
[α]_{D}²³ =+51.5^{o}(c '1.117, CHCl₃).

### Intermediate 4

### (+)-trans-5-Fluoro-2,3-dihydro-1,4-benzodioxin-2,3-dimethanol

A solution of Intermediate 3 (7.0g) in ethanol (200ml) was stirred under hydrogen at ca 25⁰ until uptake ceased. The catalyst was filtered off using a kieselguhr bed and the filter was washed with ethanol. The combined filtrate and washes were then concentrated in vacuo to a small volume and filtered through glass fibre paper. The solvent was evaporated in vacuo and the crystalline residue was triturated with petroleum ether- ether(1:1, 10ml). The crystals were collected, washed twice with petroleum ether - ether (1:1) and dried in vacuo to give the title compound (2.51g), m.p. 120-123⁰.
[α]_{D}²³ +61.35^{o} (c 1.084, EtOH).

### Intermediate 5

### (+)-trans-5-Fluoro-2,3-dihydro-1,4-benzodioxin-2,3-dimethanol, bis methanesulphonate

Intermediate 4 (3.058g) was dissolved in a mixture of dichloromethane (30ml) and triethylamine (6.0ml). The solution was cooled in ice and a solution of methanesulphonyl chloride 2.5ml) in dichloromethane (10ml) was added over 0.25h, rinsing the dropping funnel with dichloromethane (10ml). The resulting mixture was stirred at 0^{o} for a further 0.25h, and washed successively, with water, 2M hydrochloric acid, saturated sodium bicarbonate solution and brine solution (25ml portions of each). The organic mixture was then passed through phase separation paper and the pale yellow solution was evaporated in vacuo to give a gum which solidified after cooling and stirring. The solid was triturated with ether 10ml), collected by filtration, washed twice with a minimum of ether and dried in vacuo to give the title compound, m.p. 66-68⁰
[α]_{D}²³ +29.13^{o} (c 1.047, CHCl₃).

### Intermediate 6

### (+)-trans-5-Fluoro-2,3,3a,9a-tetrahydro-2-(phenylmethyl)-1H-[1,4]-benzodioxino[2,3-c]pyrrole hydrochloride

A solution of Intermediate 5 [prepared from Intermediate 4 (80g)] in dioxan (242ml) was added over 25-30min. to benzylamine (176.6ml) preheated to 109⁰ under nitrogen. After completing the addition a further portion of dioxan (57ml) was added. The mixture was distilled at atmospheric pressure until a volume of 260ml of distillate was collected. The temperature of the mixture was allowed to cool to 100⁰ over 45min. after which a mixture of dioxan (90ml) and water (173ml) was added. A further quantity of water (350ml) was added to the stirred suspension which was then warmed to 50⁰. IPE (520ml) was added and the mixture was stirred until the two phases were nearly clear. The two phases were separated and the aqueous layer was re-extracted with IPE (230ml). The two ethereal extracts were then washed sequentially with aqueous acetic acid (10% v/v, 520ml) and water (520ml). IMS (100ml) was added to the combined organic extracts and this solution was stirred and heated to reflux. Concentrated hydrochloric acid (33ml) in IMS (35ml) was then added over 20min. whilst refluxing was continued. A further portion of concentrated hydrochloric acid (10ml) in IMS (10ml) was added and the stirred suspension was cooled to ambient temperature. The solid was harvested, washed with IPE-IMS (3:1; 2x185ml), followed by IPE (185ml.) and dried in vacuo at 40⁰ to give Intermediate 6 (112.3g).
[α]_{D}²³ + 130⁰ (c 0.86, DMSO).

### Example 1

### (+)-3aR-trans-5-Fluoro-2,3,3a,9a-tetrahydro-1H-[1,4]benzodioxino [2,3,-c]pyrrole hydrochloride hemihydrate

Intermediate 6 (110.1g) in n-propanol (921ml) and distilled water (227ml) containing concentrated hydrochloric acid (1.5ml) was hydrogenated over 5% palladium on carbon (19.2g). When the uptake of hydrogen was complete, the catalyst was quickly removed by filtration through kieselguhr and the bed was washed with a solution of water (28ml) in n-propanol (213ml) and n-propanol (213ml). The combined filtrate and washes were concentrated in vacuo to 200ml and a further portion of n-propanol (56ml) was added. The mixture was again reevaporated in vacuo to a volume of 200ml and a further portion of propanol (56ml) was added whereupon the volume of the mixture was again reduced to 200ml and the vacuum was released. The mixture was stirred and heated to 45⁰and diluted with IPE (280ml) over 25 min. after which the temperature was reduced to ca. 5⁰. The mixture was then stirred at this temperature, for 2 days. The product was recovered by filtration, washed with n-propanol-IPE (1:1; 135ml) followed by IPE (135ml) and dried in vacuo at 40⁰ to give the title compound (78.34g).
[α]_{D}²³ +160⁰ (c 1.275, DMSO).

### Intermediate 7

### 1,4-Di-0-benzyl-D-threitol

This may be prepared by the method described in K.A. Nelson et al, J. Org.Chem. 1986, 51, 2721.

### Intermediate 8

### [2R-(R*,R*)]-(+)-1,4-Bis(phenylmethoxy)-2,3-butanediol, bis(4-methylbenzenesulphonate)

A solution of 1,4-di-0-benzyl-D-threitol (32.7g.) in pyridine (400ml) was cooled in ice and toluene-4-sulphonyl chloride (49.05g) was added. The reaction mixture was stirred at ca. 20⁰ for 4 days when it was cooled in ice and treated dropwise with water to destroy any unreacted toluene-4-sulphonyl chloride. After 15 min. the mixture solidified and water (1000ml) was added. The white precipitate was collected, washed with water and dried in vacuo to give the title compound, m.p. 123-125^{o}.
[α]_{D}^{22.5} +14.7(c 0.888, CHCl₃).

### Intermediate 9

### (-)-trans-5-Fluoro-2,3-dihydro-2,3-bis[(phenylmethoxy)methyl]-1, 4-benzodioxin

A solution of 3-fluorobenzene-1,2-diol (4.09g) and Intermediate 8 (23.4g) in dimethylformamide (130ml) was stirred under nitrogen for 45 min. Anhydrous caesium carbonate (13.52g) was added and the mixture was stirred at 140⁰ for 16h. After cooling, the reaction mixture was diluted with IPE (370ml) and water (320ml). The layers were separated and the aqueous phase was re-extracted with IPE (150ml and 100ml). The combined organic extracts were washed with M hydrochloric acid, brine and dried. After evaporation in vacuo the residual dark brown oil was chromatographed on Sorbsil (400g), eluting with petroleum ether-ethyl acetate (6:1) to give the title compound as a pale yellow oil (7.8g).
[α]_{D}²⁰ -47.6^{o} (c 1.28, CHCl₃).

### Intermediate 10

### (-)-trans-5-Fluoro-2,3-dihydro-1,4-benzodioxin-2,3-dimethanol

A solution of Intermediate 9 (7.65g) in methanol (100ml), containing 10% palladium on carbon (0.765g) was hydrogenolysed until hydrogen uptake ceased (4.5h). The catalyst was removed by filtration, through kieselguhr and the filtrate was evaporated in vacuo to give an off-white solid which was triturated with ether, collected by filtration and washed with ether. The white solid, thus obtained, was identified as the title compound, m.p. 121-124⁰.
[α]_{D}²⁰ -59.9^{o} (c 0.951, EtOH).

### Intermediate 11

### (-)-trans-5-Fluoro-2,3-dihydro-1,4-benzodioxin-2,3-dimethanol, bis methanesulphonate

A solution of Intermediate 10 (2.96g) in dichloromethane (30ml), containing triethylamine (6ml) was stirred at 0⁰ for 10 min and a solution of methanesulphonyl chloride (3.05ml) in dichloromethane (10ml) was added over 10 min. The mixture was then stirred at 0⁰ for 0.5h. After the addition of water (25ml) the mixture was stirred for 0.5h when the layers were separated, and the aqueous phase was reextracted with dichloromethane. The combined organic extracts were washed with 2M hydrochloric acid and aqueous sodium bicarbonate solution, dried and concentrated in vacuo. The brown gum which was obtained (5.2g) was chromatographed on Sorbsil (125g), eluting with dichloromethane-ethyl acetate (9:1), to give a pale yellow oil, which crystallised as an off-white solid. This was recrystallised from ethyl acetate-IPE to give the title compound as white crystals, m.p. 67-70⁰.
[α]_{D}²⁰ -29.8^{o} (c 1.041, CHCl₃)

### Intermediate 12

### (-)-trans-5-Fluoro-2,3,3a,9a-tetrahydro-2-phenylmethyl-1H-[1,4]-benzodioxino[2,3-c]pyrrole hydrochloride

A solution of Intermediate 11 [prepared from Intermediate 10 (74g)] in dioxan (220ml) was added over 0.5h to benzylamine (160ml) preheated to 110⁰ under nitrogen. The rate of addition was controlled to maintain the temperature of the reaction mixture between 110-120⁰. After adding a further portion of dioxan (50ml) the mixture was distilled at atmospheric pressure until 160ml of distillate was collected. The reaction mixture was then refluxed under nitrogen for 0.75h, allowed to cool to below 100⁰ and diluted over 2 min. with water (160ml). An additional portion of water (320ml) was added, followed by IPE (475ml), and the stirred mixture was maintained to ca. 50⁰ to give two clear phases. The layers were separated and the aqueous phase was re-extracted with IPE (210ml). The two organic extracts were washed sequentially with aqueous acetic acid (10% v/v, 475ml) and water (475ml). IMS (90ml) was added to the combined ethereal extracts and the stirred solution was heated to reflux. A mixture of concentrated hydrochloric acid (40ml) in IMS (45ml) was added over 15 min. and the resultant white suspension was stirred for 5.75h whilst it cooled to ambient temperature. The product was collected by filtration, washed with IPE - IMS (3:1, 3x115ml) and dried in vacuo at 40⁰ to give the title compound (103.3g).
[α]_{D}²⁰ -130⁰ (c 0.85, DMSO).

### Example 2

### (-)-3aS-trans-5-Fluoro-2,3,3a,9a-tetrahydro-1H-[1,4]-benzodioxino [2,3-c]pyrrole hydrochloride hemihydrate

A suspension of Intermediate 12 (102.5g) in n-propanol (860ml) and distilled water (210ml) containing concentrated hydrochloric acid (1.5ml) was hydrogenated at atmospheric pressure using 5% palladium on carbon catalyst. Hydrogen uptake was complete in 0.75h. The mixture was stirred for a further 20min. and the catalyst was filtered off through kieselguhr and the filter bed was washed with n-propanol-water (8:1; 225ml) followed by n-propanol (200ml). The combined filtrate and washes were reduced to a volume of 200ml and n-propanol (50ml) was added to the resultant suspension. This evaporation/addition exercise was then repeated. The solution was again reduced to a 200ml volume and IPE (260ml) was added over 1 min. with stirring. The mixture was cooled to ca. 0⁰ for 5h and the crystalline solid was harvested, washed with n-propanol-IPE (1:1; 125ml) and IPE (125ml) and dried in vacuo at 40⁰ to give the title compound (72.34g).
[α]_{D}²⁰ ·159⁰ (c 0.86, DMSO).

### Intermediate 13

### (+)-trans-5-Fluoro-2,3,3a,9a-tetrahydro-2-phenylmethyl-1H-[1,4]-benzodioxino[2,3-c]pyrrole

A homogeneous mixture of Intermediate 5(4.7g) and benzylamine (7.1ml) was heated at 130⁰, under nitrogen, for 0.5h and allowed to cool. The crystalline mass was partitioned between IPE (80ml) and water (80ml) and the organic layer was separated. The aqueous phase was re-extracted with IPE (100ml) and the two organic solutions were washed separately, with aqueous acetic acid (2.5% v/v; 2x50ml) and saturated sodium bicarbonate solution (100ml). The organic solutions were combined, passed through a phase separation paper and evaporated in vacuo to give a brown crystalline solid. This was recrystallised from IPE-petroleum ether and the solid was collected (after filtering hot to remove a trace of oil plus undissolved solid), washed twice with a minimum of IPE followed by petroleum ether and dried in vacuo to give the title compound, m.p. 87⁰. The filter paper from the hot fraction was extracted with dichloromethane and this solution was combined with the mother liquors from the foregoing crystallisation. This mixture was evaporated and the residue (1.2g) was chromatographed over Sorbsil (40g) eluting with petroleum ether -ethyl acetate (4:1). Fractions containing the title compound were combined and evaporated to afford pale yellow crystals (0.915g), m.p. 86-87⁰.
[α]_{D}²³ +73.6^{o}(c 1.10, CHCl₃).

### Example 3

### (+)-3aR-trans-5-Fluoro-2,3,3a,9a-tetrahydro-1H-[1,4]- benzodioxino-[2,3-c]pyrrolehydrochloride

Intermediate 13 (3.15g) was suspended in ethanol (150ml) and the mixture was hydrogenated at ambient temperature and pressure, using 10% palladium on carbon (0.4g), until hydrogen uptake ceased. The mixture was filtered through kieselguhr and the bed was washed with ethanol. The combined filtrate and washes were concentrated in vacuo to a low volume and filtered through glass filter paper to remove traces of carbon. Evaporation gave a pale pink crystalline solid (1.897g) which was redissolved in hot ethanol (10ml). Concentrated hydrochloric acid (0.87ml) was added to the hot solution and the resultant solution was allowed to cool. The crystals produced were collected, washed in succession with a minimum of cold ethanol and ether and dried in vacuo to give the title compound (1.27g), m.p. 235,236⁰ (sublimation at ca 220⁰).
[α]_{D}23 +159.6^{o} (c 0.871, DMSO).

### Intermediate 14

### (-)-trans-5-Fluoro-2,3,3a,9a-tetrahydro-2-phenylmethyl-1H-[1,4]- benzodioxino[2,3-c]pyrrole

A mixture of Intermediate 11 (3.65g) and benzylamine (5.4ml) was heated to 120⁰, for 20 min. After cooling, water was added and the mixture was stirred for 20min. The solid which formed was collected by filtration, then crystallised from - petroleum ether. Recrystallisation from hot IPE gave the title compound (1.57g) as pale brown crystals, m.p. 87.5-88.5⁰.
[α]_{D}²⁰ -73.8⁰(c 0.92, CHCl₃).

Chromatography of the combined mother liquors over Sorbsil (25g), eluting with petroleum ether-ethyl acetate (4:1) afforded a further quantity of the title compound (0.54g) as pale yellow crystals.

### Example 4

### (-)-3aS-trans-5-Fluoro-2,3,3a,9a-tetrahydro-1H-[1,4]-benzodioxino[2,3-c]-pyrrole hydrochloride

A solution of Intermediate 14 (2.35g) in methanol (130ml) was stirred under hydrogen, in the presence of 10% palladium on carbon until hydrogen uptake was complete. The catalyst was removed by filtration through kieselguhr and the filtrate was concentrated in vacuo to give a grey solid. This was dissolved in methanol (10ml) and concentrated hydrochloric acid (1ml) was added. After 0.25h, the solid (1.2g) which had formed was collected and was recrystallised from hot methanol to give the title compound (0.9g) as off-white crystals, m.p. 238-240⁰.
[α]_{D}²⁰-159.4^{o} (c 0.847, DMSO).

## Claims

1. A compound (+)-3aR-trans-5-fluoro-2,3,3a,9a- tetrahydro-1H-[1,4]-benzodioxino[2,3-c]pyrrole or a physiologically acceptable salt or hydrate thereof.

2. A compound (-)-3aS-trans-5-fluoro-2,3,3a,9a-tetrahydro-1H-[1,4]-benzodioxino[2,3-c]pyrrole or a physiologically acceptable salt or hydrate thereof.

3. A compound according to claim 1 or claim 2 in the form of a hydrochloride salt.

4. A compound according to claim 3 in the form of a hemihydrate.

5. A compound according to any one of claims 1 to 4 or a physiologically acceptable salt or hydrate thereof for use in medicine, especially for use in the treatment of a condition that is ameliorated by administration of a selective α₂-adrenoreceptor antagonist.

6. A compound according to any one of claims 1 to 4 or a physiologically acceptable salt or hydrate thereof for the manufacture of a medicament for the treatment of a condition that is ameliorated by administration of a selective α₂-adrenoreceptor antagonist.

7. A pharmaceutical formulation comprising a compound according to any one of claims 1 to 4 or a physiologically acceptable salt or hydrate thereof together with a physiologically acceptable carrier or excipient.

8. A method for the preparation of a compound according to any one of claims 1 to 4 or a physiologically acceptable salt or hydrate thereof, which comprises
(A) aminating an optically active trans-benzodioxin derivative of general formula (II) 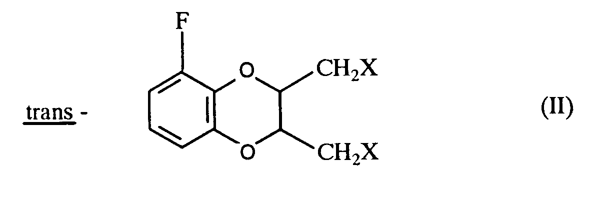 where X is a leaving group, such as a halogen atom or a hydrocarbylsulphonyloxy group,
with ammonia, aqueous ammonia or an amine of formula RNH₂ where R is a nitrogen protecting group,
followed if necessary, by removal of the nitrogen protecting group; or
(B) deprotecting an optically active derivative of a compound according to any one of claims 1 to 4 in which the nitrogen atom is protected by a nitrogen protecting group.

## Claims (Claims for the following Contracting State(s): ES, GR)

1. A method for the preparation of an optically active compound of general formula I 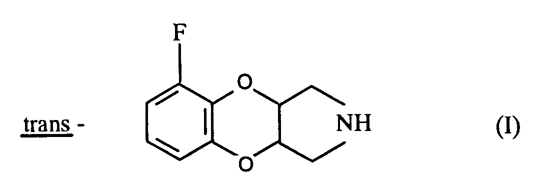 or a physiologically acceptable salt or hydrate thereof, which comprises
(A) aminating an optically active trans-benzodioxin derivative of general formula (II) 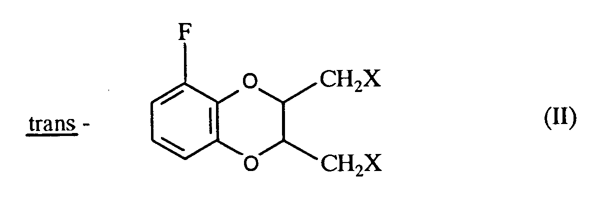 where X is a leaving group, such as a halogen atom or a hydrocarbylsulphonyloxy group,
with ammonia, aqueous ammonia or an amine of formula RNH₂ where R is a nitrogen protecting group,
followed if necessary, by removal of the nitrogen protecting group; or
(B) deprotecting an optically active derivative of a compound of general formula I in which the nitrogen atom is protected by a nitrogen protecting group.

2. A process according to claim I characterised in that the optically active compound of general formula I is (+)-3aR-trans-5-fluoro-2,3,3a,9a- tetrahydro-1H-[1,4]-benzodioxino[2,3-c]pyrrole or a physiologically acceptable salt or hydrate thereof, especially the hydrochloride salt thereof.

3. A process according to claim I characterised in that the optically active compound of general formula I is (-)-3aS-trans-5-fluoro-2,3,3a,9a- tetrahydro-1H-[1,4]-benzodioxino[2,3-c]pyrrole or a physiologically acceptable salt or hydrate thereof, especially the hydrochloride salt thereof.

4. A process for the preparation of a pharmaceutical formulation comprising admixing an optically active compound of general formula I 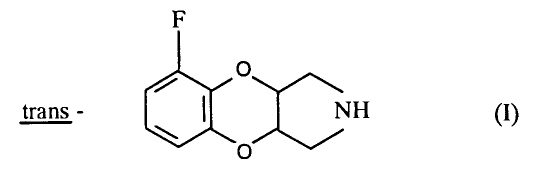 or a physiologically acceptable salt or hydrate thereof
with a physiologically acceptable carrier or excipient.
